# EUROPEAN PATENT SPECIFICATION

(11) **EP 1 915 466 B1**
(45) Date of publication and mention of the grant of the patent: **24.11.2010**
(21) Application number: 06801832.4
(22) Date of filing: 18.08.2006
(51) Int. Cl.: C12Q 1/68, G01N 33/543

(54) **METHODS FOR PREPARING HYBRID SUBSTRATES COMPRISING DNA AND ANTIBODIES AND USES THEREOF**
VERFAHREN ZUR HERSTELLUNG VON HYBRIDSUBSTRATEN MIT DNA UND ANTIKÖRPERN SOWIE VERWENDUNGEN DAVON
PROCEDES DE PREPARATION DE SUBSTRATS HYBRIDES COMPRENANT DE L'ADN ET DES ANTICORPS ET UTILISATIONS DE CEUX-CI

(30) Priority: 19.08.2005 US 709613 P
(43) Date of publication of application: 30.04.2008
(73) Proprietor: Nanosphere, Inc., Northbrook, Illinois 60062 (US)
(72) Inventor: STORHOFF, James, J., Evanston, IL 60202 (US); SENICAL, Michael, J., Chicago, IL 60645 (US)
(74) Representative: Grund, Martin
(86) International application number: PCT/US2006/032301
(87) International publication number: WO 2007/024676

(56) References cited:
- WO-A-2004/081575
- WO-A2-02/079490
- WO-A2-2004/076678
- WO-A2-2005/003394
- WO-A2-2005/113817
- US-A1- 2004 115 794
- TEMPLIN M F ET AL: "PROTEIN MICROARRAY TECHNOLOGY" DDT - DRUG DISCOVERY TODAY, ELSEVIER SCIENCE LTD, GB, vol. 7, no. 15, 1 August 2002 (2002-08-01), pages 815-822, XP001152984 ISSN: 1359-6446
- TAUSSIG M J ET AL: "Progress in antibody arrays" TARGETS, ELSEVIER, vol. 2, no. 4, 1 August 2003 (2003-08-01), pages 169-176, XP004886670 ISSN: 1477-3627
- COLLETT JAMES R ET AL: "Functional RNA microarrays for high-throughput screening of antiprotein aptamers" ANALYTICAL BIOCHEMISTRY, vol. 338, no. 1, 1 March 2005 (2005-03-01), pages 113-123, XP002413233 ISSN: 0003-2697
- HOPPE-SEYLER FELIX ET AL: "Peptide aptamers: Powerful new tools for molecular medicine" JOURNAL OF MOLECULAR MEDICINE (BERLIN), vol. 78, no. 8, 2000, pages 426-430, XP002413234 ISSN: 0946-2716
- LOPEZ M F ET AL: "Protein micro- and macroarrays: digitizing the proteome" JOURNAL OF CHROMATOGRAPHY B: BIOMEDICAL SCIENCES & APPLICATIONS, ELSEVIER, AMSTERDAM, NL, vol. 787, no. 1, 5 April 2003 (2003-04-05), pages 19-27, XP004415587 ISSN: 1570-0232
- BRODY E N ET AL: "The use of aptamers in large arrays for molecular diagnostics" MOLECULAR DIAGNOSIS 1999 UNITED STATES, vol. 4, no. 4, 1999, pages 381-388, XP005533507 ISSN: 1084-8592
- WALTER GERALD ET AL: "High-throughput protein arrays: Prospects for molecular diagnostics" TRENDS IN MOLECULAR MEDICINE, vol. 8, no. 6, June 2002 (2002-06), pages 250-253, XP002413235 ISSN: 1471-4914
- WENG S ET AL: "GENERATING ADDRESSABLE PROTEIN MICROARRAYS WITH PROFUSION COVALENT MRNA-PROTEIN FUSION TECHNOLOGY" PROTEOMICS, WILEY - VCH VERLAG, WEINHEIM, DE, vol. 2, no. 1, January 2002 (2002-01), pages 48-57, XP009010180 ISSN: 1615-9853
- NAM JWA-MIN ET AL: "Nanoparticle-based bio-bar codes for the ultrasensitive detection of proteins." SCIENCE (WASHINGTON D C), vol. 301, no. 5641, 26 September 2003 (2003-09-26), pages 1884-1886, XP002403223 ISSN: 0036-8075 cited in the application
- NAM JWA-MIN ET AL: "Bio-bar-code-based DNA detection with PCR-like sensitivity." JOURNAL OF THE AMERICAN CHEMICAL SOCIETY, vol. 126, no. 19, 19 May 2004 (2004-05-19), pages 5932-5933, XP002403224 ISSN: 0002-7863

## Description

### FIELD OF THE INVENTION

The invention relates to methods of detecting target analytes in a sample comprising detecting binding of a target analyte to capture probes on a substrate, wherein some of the capture probes comprise antibodies and other capture probes comprise aptamers, and all of the capture probes are bound to the substrate. The invention further relates to substrates that have antibodies and capture oligonucleotides bound thereto.

### BACKGROUND OF THE INVENTION

Detection of analytes is important for both molecular biology research and medical applications. Diagnostic methods based on fluorescence, mass spectroscopy, gel electrophoresis, laser scanning and electrochemistry are now available for identifying a variety of protein structures (Pandey and Mann, 2000, Nature 405:837-846; Fields and Song, 1989, Nature 340:245-246; Ijksma et al., 2001, Anal. Chem. 73:901-907; Service, 2000, Science 287:2136-2138). Antibody-based reactions are widely used to identify the genetic protein variants of blood cells, diagnose diseases, localize molecular probes in tissue, and purify molecules or effect separation processes (Zole, Monoclonal Antibodies, Springer-Verlag, New York, 2000, p.1-5). For medical diagnostic applications (*e.g*. malaria and HIV), antibody tests such as the enzyme-linked immunosorbent assay, Western blotting, and indirect fluorescent antibody tests are extremely useful for identifying single target protein structures (Butler, 2000, Immunoassay 21:165-209; Herbrink et al., 1991, Tech. Diagn. Pathol. 2:1-19).

For DNA detection methods, many assays have been developed using radioactive labels, molecular fluorophores, chemiluminescence schemes, electrochemical tags, and most recently, nanostructure-based labels (Nicewarner-Pena et al., 2001, Science 294, 137; Han et al., 2001, Nat. Biotechnol. 19, 631; Zhao et al., 2003, J. Am. Chem. Soc. 125, 11474; Lortie et al., 1991, J. Clin. Microbiol. 29, 2250; Zeph et al., 1991, Curr. Microbiol. 22, 79; Yu et al., 2001, J. Am. Chem. Soc., 123, 11155; Taton et al., 2000, Science 289, 1757; Park et al., 2002, Science 295, 1503; Cao et al., 2002, Science 297,1536; Saghatelian et al., 2003, J. Am. Chem. Soc. 125, 344).

In certain instances, it is also desirable to detect different types of analytes, such as nucleic acid molecules and proteins, in a single sample. Currently, the ability to detect these different types of analytes is limited by the need to immobilize nucleic acid and antibodies/proteins on separate substrates, requiring multiple tests or substrates if different types of capture elements (*e.g*., antibodies and nucleic acid aptamers) are required for analyte detection. Thus, there is a need in the art for efficient and rapid assays that can detect multiple analytes in a single sample, particularly where the analytes are different types of molecules, for example one type of analyte is a protein and another type is a nucleic acid molecule. COLLETT JAMES R ET AL disclose functional RNA microarrays for high-throughput screening of antiprotein aptamers (ANALYTICAL BIOCHEMISTRY, vol. 338, no. 1,1 March 2005 (2005-03-01), pages 113-123). WO 2004/076678 relates to a screening assay using mass spectroscopy. WO 2004/076678 describes a translucent solid matrix assay device for microarray analysis.

### SUMMARY OF THE INVENTION

The invention relates to subject matter as defined in the appended claims. Disclosed are methods for detecting at least one target analyte in a sample, the target analyte having at least two binding sites, the method comprising the steps of: (a) providing a substrate having a first type of capture probe that comprises an antibody and a second type of capture probe that comprises an aptamer bound thereto, wherein each type of capture probe can bind to a first binding site of a specific target analyte; (b) optionally providing at least one type of detector probe that can bind to a second binding site of the target analyte; (c) contacting the sample with the substrate and the detector probe under conditions that are effective for the binding of each type of capture probe to the first binding site of the target analyte and optionally the binding of the detector probe to the second binding site of the target analyte to form a complex; (d) washing the substrate to remove unbound materials; and (e) detecting for the presence or absence of the complex, wherein the presence or absence of the complex is indicative of the presence or absence of the specific target analyte in the sample.

In certain aspects, a sample is first contacted with the detector probe so that a target analyte present in the sample binds to the detector probe, and the target analyte bound to the detector probe is then contacted with the substrate so that the target analyte binds to at least one type of capture probe on the substrate. Alternatively, a sample is first contacted with the substrate so that a target analyte present in the sample binds to at least one type of capture probe, and the target analyte bound to the capture probe is then contacted with the detector probe so that the target analyte binds to the detector probe. In other aspects, a sample, the detector probe and the capture probes on a substrate are contacted simultaneously.

A captured target-detector probe complex can be detected by photonic, electronic, acoustic, opto-acoustic, gravity, electro-chemical, electro-optic, mass-spectrometric, enzymatic, chemical, biochemical, or physical means.

In certain aspects, a target analyte is a protein and the detector probe is a nanoparticle probe that comprises: (a) antibodies that bind the target analyte; (b) aptamers that bind the target analyte; or (c) a mixture of antibodies and aptamers that bind the target analyte. Disclosed are substrates that comprise both antibodies and aptamers bound thereto. The antibodies and aptamers can be specific for the same target analyte (*i.e.,* both bind to the same target analyte) or each can be specific for a different target analyte (*i.e.*, antibodies could bind specifically to one target analyte and the aptamers could bind to a different target analyte). In addition, the invention provides substrates that comprise capture probes (such as oligonucleotides, antibodies and/or aptamers) and capture oligonucleotides bound thereto. The capture probes can be specific for proteins or nucleic acid molecules. In certain aspects, the substrate can comprise multiple types of capture probes, some that bind proteins and some that bind nucleic acid molecules, thereby enabling the detection of proteins and nucleic acid molecules on a single substrate in a single assay.

These and other embodiments of the invention will become apparent in light of the detailed description and examples below.

### BRIEF DESCRIPTION OF THE DRAWINGS

Figure 1 shows a schematic illustration of assay for detecting a single or multiple protein targets.
Figure 2 shows detection of human IgE using aptamer/antibody slides of the invention.
Figure 3 relates to subject matter not encompassed by the invention and shows a schematic illustration of a barcode assay on hybrid antibody/DNA slides.

### DETAILED DESCRIPTION OF THE PREFERRED EMBODIMENTS

Unless otherwise required by context, singular terms shall include pluralities and plural terms shall include the singular.

As utilized in accordance with the present disclosure, the following terms, unless otherwise indicated, shall be understood to have the following meanings:

As used herein, a "nucleic acid sequence," a "nucleic acid molecule," or nucleic acids" refers to one or more oligonucleotides or polynucleotides as defined herein.

The term "polynucleotide" as referred to herein means single-stranded or double-stranded nucleic acid polymers of at least 10 bases in length. In certain embodiments, the nucleotides comprising the polynucleotide can be ribonucleotides or deoxyribonucleotides or a modified form of either type of nucleotide. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "oligonucleotide" referred to herein includes naturally occurring, and modified nucleotides linked together by naturally occurring, and/or non-naturally occurring oligonucleotide linkages. Oligonucleotides are a polynucleotide subset comprising members that are generally single-stranded and have a length of 200 bases or fewer. In certain embodiments, oligonucleotides are 10 to 60 bases in length. In certain embodiments, oligonucleotides are 10, 11, 12, 13, 14, 15, 16, 17, 18, 19, or 20 to 40 bases in length. Oligonucleotides may be single stranded or double stranded, *e.g*. for use in the construction of a gene mutant. Oligonucleotides of the invention may be sense or antisense oligonucleotides with reference to a protein-coding sequence.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like. *See, e.g.,* LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, OLIGONUCLEOTIDES AND ANALOGUES: A PRACTICAL APPROACH, pp. 87-108 (F. Eckstein, Ed.), Oxford University Press, Oxford England; Stec et al., U.S. Pat. No. 5,151,510; Uhlmann and Peyman, 1990, Chemical Reviews, 90:543. An oligonucleotide can include a detectable label to enable detection of the oligonucleotide or hybridization thereof.

A "substrate" or an "addressable substrate" used in a method of the invention can be any surface capable of having antibodies, aptamers, oligonucleotides, or analytes bound thereto. Such surfaces include, but are not limited to, a nanoparticle, a thin film, magnetic beads, or any material comprising glass, metal, plastic, or materials coated with a functional group designed for binding of antibodies, aptamers, oligonucleotides, or analytes. The coating may be thicker than a monomolecular layer; in fact, the coating could involve porous materials of sufficient thickness to generate a porous 3-dimensional structure into which the antibodies, aptamers, oligonucleotides, or analytes can diffuse and bind to the internal surfaces.

Binding of capture probes, such as antibodies, aptamers, and oligonucleotides, to a substrate can be accomplished by any methods known to those of skill in the art and as described, for example, in U.S. Patent Application No. 11/124609, filed May 6, 2005.

The term "capture probe" as used herein can refer to an aptamer or an antibody, or a complex that comprises aptamers and/or antibodies (*e.g*. a particle having aptamers and/or antibodies bound thereto).

A "detection probe" or "detector probe" of the invention can be any carrier to which one or more detection aptamers, antibodies, or oligonucleotides can be attached, wherein the one or more detection aptamers, antibodies, or oligonucleotides can comprise a configuration that binds a specific target analyte. Detector probes can comprise fluorophores or phosphors (including, for example, particles doped with phosphors or fluorophores), quantum dots, enzyme conjugates (such as horseradish peroxidase), or antibody-DNA conjugates (which can be used, for example, in immunoPCR or rolling circle amplification detection assays). The carrier itself may serve as a label, or may contain or be modified with a detectable label, or the detection aptamers, antibodies, or oligonucleotides may carry such labels. Carriers that are suitable for the methods of the invention include, but are not limited to, microparticles, nanoparticles, quantum dots, dendrimers, semi-conductors, beads, up- or down-converting phosphors, large proteins, lipids, carbohydrates, or any suitable inorganic or organic molecule of sufficient size, or a combination thereof.

As used herein, the terms "label" or "detection label" refers to a detectable marker that may be detected by photonic, electronic, opto-electronic, magnetic, gravity, acoustic, enzymatic, or other physical or chemical means. The term "labeled" as used herein refers to incorporation of such a detectable marker (*e.g*., by incorporation of a radiolabeled nucleotide, or attachment to aptamers, antibodies, or oligonucleotides of a detectable marker).

A "sample" as used herein refers to any quantity of a substance that comprises or potentially comprises target analytes, such as proteins or nucleic acids, and that can be used in a method of the invention. For example, the sample can be a biological sample or can be extracted from a biological sample derived from humans, animals, plants, fungi, yeast, bacteria, viruses, tissue cultures or viral cultures, or a combination of the above. They may contain or be extracted from solid tissues (e.g. bone marrow, lymph nodes, brain, skin), body fluids (e.g. serum, blood, urine, sputum, seminal or lymph fluids), skeletal tissues, or individual cells. Alternatively, the sample can comprise purified or partially purified analytes, such as proteins or nucleic acid molecules, and, for example, buffers and/or reagents that are used to generate appropriate conditions for successfully performing a method of the invention.

The term "analyte" or "target analyte" refers to a compound or composition to be detected or assayed by the method of the invention. Typical analytes may include, but are not limited to proteins, peptides, nucleic acid segments, small molecules, cells, microorganisms and fragments and products thereof, or any substance for which attachment sites, binding members or receptors (such as antibodies) can be developed. The analytes have at least one binding site, preferably at least two binding sites (*e.g*., epitopes) that can be targeted by a capture probe and/or a detection probe (*e.g*. antibodies or aptamers or both).

An analyte may be a molecule found directly in a sample such as a body fluid from a host. The sample can be examined directly or may be pretreated to render the analyte more readily detectible. Furthermore, the analyte of interest may be determined by detecting an agent probative of the analyte of interest such as a specific binding pair member complementary to the analyte of interest, whose presence will be detected only when the analyte of interest is present in a sample. Thus, the agent probative of the analyte becomes the analyte that is detected in an assay. The body fluid can be, for example, urine, blood, plasma, serum, saliva, semen, stool, sputum, cerebral spinal fluid, tears, mucus, and the like.

The term "specific binding" refers to the specific recognition of one of two different molecules for the other compared to substantially less recognition of other molecules. Generally, the molecules have areas on their surfaces or in cavities giving rise to specific recognition between the two molecules. Exemplary of specific binding are antibody-antigen interactions, enzyme-substrate interactions, polynucleotide interactions, and so forth.

The term "non-specific binding" refers to the binding between molecules that is relatively independent of specific surface structures. Non-specific binding may result from several factors including hydrophobic interactions between molecules.

The term "target recognition element" refers to any binding agent that can be used bind a target analyte. For example, a target recognition element can be an antibody, an epitope binding antibody fragment (such as a Fab), an Affibody^{®} (Affibody AB, Bromma, Sweden), nanobody molecule, single-chain antibody fragment (scFv), or peptides.

The term "antibody" refers to an immunoglobulin which specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')₂, Fab', and the like. In addition, aggregates, polymers, and conjugates of immunoglobulins or their fragments can be used where appropriate so long as binding affinity for a particular molecule is maintained.

Target analytes such as proteins, polypeptides, fragments, variants, and derivatives may be used to prepare antibodies using methods known in the art. Thus, antibodies and antibody fragments that bind to target analytes may be used in the methods of the invention as a capture probe on a substrate when aptamer detection probes are used, as a detection probe when aptamer is used as a capture probe on a substrate, or as detection probes in combination with an aptamer detection probe. Antibodies may be polyclonal, monospecific polyclonal, monoclonal, recombinant, chimeric, humanized, fully human, single chain and/or bispecific.

Polyclonal antibodies directed toward a target analyte generally are raised in animals (e.g., rabbits or mice) by multiple subcutaneous or intraperitoneal injections of an antigen and an adjuvant. It may be useful to conjugate a target analyte protein, polypeptide, or a variant, fragment or derivative thereof to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-target analyte antibody titer.

Monoclonal antibodies directed toward target analytes are produced using any method that provides for the production of antibody molecules by continuous cell lines in culture. Examples of suitable methods for preparing monoclonal antibodies include hybridoma methods of Kohler, et al., Nature 256:495-97 (1975), and the human B-cell hybridoma method, Kozbor, J. Immunol. 133:3001 (1984); Brodeur et al., Monoclonal Antibody Production Techniques and Applications 51-63 (Marcel Dekker 1987).

The term "aptamers" as used herein refers to nucleic acids (typically DNA, RNA or oligonucleotides) that emerge from in vitro selections or other types of aptamer selection procedures well known in the art (e.g. bead-based selection with flow cytometry or high density aptamer arrays) when the nucleic acid is added to mixtures of molecules. Ligands that bind aptamers include but are not limited to small molecules, peptides, proteins, carbohydrates, hormones, sugar, metabolic byproducts, cofactors, drugs and toxins. Aptamers of the invention are preferably specific for a particular analyte. Aptamers can have diagnostic, target validation and therapeutic applications. The specificity of the binding is defined in terms of the dissociation constant Kd of the aptamer for its ligand. Aptamers can have high affinity with Kd range similar to antibody (pM to nM) and specificity similar/superior to antibody (Tuerk and Gold, 1990, Science, 249:505; Ellington and Szostak, 1990, Nature 346:818). An aptamer will typically be between 10 and 300 nucleotides in length. RNAs and DNAs aptamers can be generated from in vitro selection experiments such as SELEX (Systematic Evolution of Ligands by Exponential Enrichment). Examples of aptamer uses and technology are PhotoSELEX™ and Riboreporters™. Aptamers, their uses, and manufacture are described, for example, in U.S. Patent Nos. 5,840,867, 6,001,648, 6225,058, 6,207,388 and U.S. Patent publication 20020001810.

Aptamers configured to bind to specific target analytes can be selected, for example, by synthesizing an initial heterogeneous population of oligonucleotides, and then selecting oligonucleotides within the population that bind tightly to a particular target analyte. Once an aptamer that binds to a particular target molecule has been identified, it can be replicated using a variety of techniques known in biological and other arts, for example, by cloning and polymerase chain reaction (PCR) amplification followed by transcription.

The synthesis of a heterogeneous population of oligonucleotides and the selection of aptamers within that population can be accomplished using a procedure known as the Systematic Evolution of Ligands by Exponential Enrichment or SELEX. The SELEX method is described in, for example, Gold et al., U.S. Patent Nos. 5,270,163 and 5,567,588; Fitzwater et al., "A SELEX Primer," Methods in Enzymology, 267:275-301 (1996); and in Ellington and Szostak, "In Vitro Selection of RNA Molecules that Bind Specific Ligands," Nature, 346:818-22. For example, a heterogeneous DNA oligomer population can be synthesized to provide candidate oligomers for the *in vitro* selection of aptamers. The initial DNA oligomer population is a set of random sequences 15 to 100 nucleotides in length flanked by fixed 5' and 3' sequences 10 to 50 nucleotides in length. The fixed regions provide sites for PCR primer hybridization and, in one implementation, for initiation of transcription by an RNA polymerase to produce a population of RNA oligomers. The fixed regions also contain restriction sites for cloning selected aptamers. Many examples of fixed regions can be used in aptamer evolution. See, *e.g*., Conrad et al., "In Vitro Selection of Nucleic Acid Aptamers That Bind Proteins," Methods in Enzymology, 267:336-83 (1996); Ciesiolka et al., "Affinity Selection-Amplification from Randomized Ribooligonucleotide Pools," Methods in Enzymology, 267:315-35 (1996); and Fitzwater et al., "A SELEX Primer," Methods in Enzymology, 267:275-301 (1996).

Aptamers are selected in a 5 to 100 cycle procedure. In each cycle, oligomers are bound to the target molecule, purified by isolating the target to which they are bound, released from the target, and then replicated by 20 to 30 generations of PCR amplification. Alternative methods for producing aptamers well known in the art also can be used, including bead-based selection or microarray-based selection.

Various oligomers can be used for aptamer selection, including, but not limited to, 2'-fluoro-ribonucleotide oligomers, NH₂-substituted and OCH₃-substituted ribose aptamers, and deoxyribose aptamers. RNA and DNA populations are equally capable of providing aptamers configured to bind to any type of target molecule. Within either population, the selected aptamers occur at a frequency of 10⁹ to 10¹³, see Gold et al., "Diversity of Oligonucleotide Functions," Annual Review of Biochemistry, 64:763-97 (1995), and most frequently have nanomolar binding affinities to the target, affinities as strong as those of antibodies to cognate antigens. See Griffiths et al., EMBO J., 13:3245-60 (1994).

Using 2'-fluoro-ribonucleotide oligomers is likely to increase binding affinities ten to one hundred fold over those obtained with unsubstituted ribo- or deoxyribo-oligonucleotides (see Pagratis et al., "Potent 2'-amino and 2' fluoro 2'deoxyribonucleotide RNA inhibitors of keratinocyte growth factor" Nature Biotechnology, 15:68-73). Such modified bases provide additional binding interactions and increase the stability of aptamer secondary structures. These modifications also make the aptamers resistant to nucleases, a significant advantage for real world applications of the system. See Lin et al., "Modified RNA sequence pools for in vitro selection" Nucleic Acids Research, 22:5229-34 (1994); and Pagratis, et al., "Potent 2'-amino and 2' fluoro 2'deoxyribonucleotide RNA inhibitors of keratinocyte growth factor" Nature Biotechnology, 15:68-73.

The aptamers may include suitable modifications that would allow the aptamer to be attached or bound to a substrate. Suitable, but non-limiting modifications include functional groups such as thiols, amines, carboxylic acids, maleimide, and dienes. Other methods such as hapten interactions may be used. Examples of hapten interactions include, but are not limited to, strepatavidin-biotin, x-biotin-biotin, x-fluorescein/fluorescein and other hapten pairs well known in the art. The aptamers can be prepared by any suitable means, including chemical synthesis and chemical synthesis on solid support.

The aptamers used in the methods of the invention may include an oligonucleotide tail. The term "oligonucleotide tail" refers to a synthetic oligonucleotide extension of the aptamer. This extension may be created during the synthesis of the aptamer or may be added to the 3' or 5' end of the aptamer using any suitable means including chemical or enzymatic means. It is important to note that this extension is added after the aptamer sequence has been selected. Thus, it does not present a part o the sequence that determines the binding activity of the aptamer. The extension is generally single-stranded and has a length of about 10 to 60 bases. In certain embodiments, the oligonucleotide are 10, 11, 12, 13, 14, 15, 16, 17, 18, 19, and 20 to 40 bases in length. The oligonucleotide tail may be any suitable length and sequence that does not interfere with the ability of the aptamer to bind to its target. The oligonucleotide tail has a predetermined sequence, allowing for modification of the aptamer to include any desired label by hybridizing an labeled oligonucleotide, e.g., a fluorophore labeled oligonucleotide, having a sequence that is complementary to at least a portion of the oligonucleotide tail.

In certain embodiments, an aptamer can be bound to a substrate or a particle (*e.g*. a nanoparticle or microparticle) and comprise a configuration that can locate (*i*.*e*. bind in a sample) a target analyte, thereby causing a target non-nucleic acid analyte to be attached to the substrate or particle via the aptamer upon binding.

In other embodiments, an aptamer can comprise an oligonucleotide tail and a second linker oligonucleotide having a sequence that is complementary to at least a portion of a sequence of the oligonucleotide tail, said second oligonucleotide having an optional label. The oligonucleotide tail advantageously allows for multiplexing, *e.g*., the attaching of different oligonucleotide probes labeled with different detection moieties such as fluorophores, dendrimers, radiolabels, enzymes, and the like. The aptamer probe having the oligonucleotide tail is broadly useful in a variety of assays for detecting target analytes, including direct or indirect sandwich assays, as described, for example, in US Patent Application No. 11/121165, filed May 3, 2005.

In certain embodiments, a "barcode probe" is used in methods of the invention. As used herein, a "barcode probe" is a complex comprising a DNA barcode bound to a particle (*e.g*. a nanoparticle or microparticle). As used herein, the term "barcode", "biochemical barcode" "biobarcode", "barcodes DNA", "DNA barcode", "reporter barcode", "reporter barcode DNA", etc. are all interchangeable with each other and have the same meaning. The DNA barcode may be a nucleic acid such as deoxynucleic acid or ribonucleic acid. Preferably, the DNA barcode is an oligonucleotide of a predefined sequence. If desired, the DNA barcode may be labeled, for instance, with biotin, a radiolabel, or a fluorescent label. The DNA barcode preferably can bind to a portion of a target analyte and to a portion of a detector oligonucleotide, thereby forming a complex that is detectable.

DNA barcodes can be directly bound to particles or can be bound to the particles through any attachment moiety, such as oligonucleotides bound to the particle that can be hybridized to at least a portion of the DNA barcodes, thereby binding the barcode to the particle. DNA barcodes can be released from the particles by exposing the barcodes to conditions under which the DNA barcodes will dehybridize from oligonucleotides by which the barcodes were attached to the particles.

Alternatively, DNA barcodes can be released from the particles to which they are attached by a chemical releasing agent that will disrupt binding of the barcode to a surface as described above and elsewhere in this disclosure. Such agents, as described herein, include, but are not limited to, any molecule.that will preferentially bind to a microparticle through a thiol link such as other thiol- or disulfide-containing molecules, dithiothreitol (DTT), dithioerythritol (DTE), mercaptoethanol and the like, and reducing agents such as sodium borohydride that will cleave a disulfide linkage thereby releasing DNA barcodes from the microparticles to which they are attached.

As described herein, a barcode can be labeled with a detectable reporter group. Suitable reporter groups include, but are not limited to, a fluorophore, a chromophore, a redox-active group, a group with an electrical signature, radioactive group, a catalytic group, or Raman label.

The Raman labels can be any one of a number of molecules with distinctive Raman scattering spectra. Unlike the enzymes used in enzyme immunoassays, these label species can be stable, simple, inexpensive molecules which can be chemically modified as required. The following attributes enhance the effectiveness of the label in this application: (a) A strong absorption band in the vicinity of the laser excitation wavelength (extinction coefficient near 10⁴; (b) A functional group which will enable covalent attachment to a specific binding member; (c) Photostability; (d) Sufficient surface and resonance enhancement to allow detection of analyte in the subnanogram range; (e) Minimal interference in the binding interaction between the labeled and unlabeled specific binding members; (f) Minimal exhibition of strong fluorescence emission at the excitation-wavelength used; (g) A relatively simple scattering pattern with a few intense peaks; and/or (h) Labels with scattering patterns which do not interfere with each other so several indicator molecules may be analyzed simultaneously.

The following is a listing of some, but not all potential candidates for these Raman-active label: 4-(4-Aminophenylazo)phenylarsonic acid monosodium salt, arsenazo I, basic fuchsin, Chicago sky blue, direct red 81, disperse orange 3, HABA (2-(4-hydroxyphenylazo)-benzoic acid), erythrosin B, trypan blue, ponceau S, ponceau SS, 1,5-difluoro-2,4-dinitrobenzene, cresyl violet and p-dimethylaminoazobenzene. The chosen labels may be covalently attached to the specific binding members of interest or attached or associated with.

Multiple Raman labels may be bound to a particle to provide a multicoding Raman labels for indexing different particles. Thus, the invention includes a reagent which has multiple Raman dyes and a specific binding substance, such as DNA, RNA, antibody, antigen, small molecule bound to the particle. For particle-based detection probes, the Raman labels or dyes can be attached directly or indirectly to the particle. The Raman label can be modified with a functional group, e.g., a thiol, amine, or phosphine that can bind to the surface of the particle such as a metallic nanoparticle. If desired, the Raman dye can be further functionalized with a molecule such as oligonucleotides (e.g., polyadenosine, polythymidine) for enhanced nanoparticle stability or with a specific binding pair member (such as an oligonucleotide having a sequence that is complementary to at least a portion of a nucleci acid target or a receptor for a particular ligand). Alternatively, the Raman label can be conjugated with a molecule or any linker, e.g., polyA or polyT oligonucleotide, that bears a functional group for binding to the particle. Raman labels can be detected as described, for example, in US Patent Application Serial No. 10/431,341 filed May 7, 2003.

The DNA biobarcode can be optionally amplified after being released, *e.g*., PCR amplification, and detected by any suitable means, such as a sandwich assay. Preferably, the DNA biobarcode is detected using a sensitive nanoparticle-based detection system using arrayed substrate and silver amplification as described, for example, in U.S. Patent No. 6,750,016 and U.S. Patent Application No. 10/877,750, filed June 25, 2004. Alternatively, the DNA biobarcode can be biotinylated, radioactively labeled, or fluorescently labeled, or any other suitable detection label. A labeled DNA biobarcode can be detected by any suitable means, including solution-based fluorometry.

For a description of the biobarcode assay, see U.S. serial no. 10/877,750, filed June 25, 2004. The biobarcode amplification assay typically involves two types of particles, a magnetic microparticle (MMP) functionalized with a group that has an affinity for a target of interest and a nanoparticle functionalized with a second group that has an affinity for the same target along with oligonucleotides (barcode DNA) that can act as reporter groups for the target of interest. When the target is a protein, the recognition agent on the magnetic particle is typically a monoclonal antibody, but may be an aptamer, and the recognition agent on the gold nanoparticle is a polyclonal or monoclonal antibody, but preferably it is an aptamer, that recognizes an epitope distinct from the one on the antibody on the magnetic particle. In the biobarcode assay, the MMP probes can be added to a solution containing the protein target of interest. After the MMP probes have been given a chance to react with target, the nanoparticle probes with barcode DNA are added to form a sandwich structure with the MMP probes that have captured target. A suitable separation technique, e.g., a magnetic field, may be used to separate such sandwich complexes from the test solution, and the supernatant is discarded. Dehybridization of the barcode DNA followed by microarray detection with gold nanoparticle probes allows one to identify the barcode sequences and quantify the amount of protein target in the test solution. In certain embodiments, the detection of the DNA barcodes occurs on the same substrate that is used to capture the target analyte, as described herein. Alternatively, the DNA barcodes bound to the nanoparticles can be further modified with any suitable label (such as a fluorophore) and detected by another suitable means such fluorophore detection methods. Thus, released DNA biobarcodes can be detected by any suitable means, depending in part on whether the biobarcodes were labeled or not.

In certain embodiments, labels that can be used as a detection label of the invention allow detection by photonic, electronic, acoustic, opto-acoustic, gravity, electro-chemical, electro-optic, mass-spectrometric, enzymatic, chemical, biochemical, or physical means. Representative examples include fluorescent, luminescent, phosphorescent, or radioactive detection labels, a quantum dot, a nanoparticle, a dendrimer, a molecular aggregate or a bead, a nanoparticle, and an oligonucleotide having a known sequence. The oligonucleotide is designed to be amplified by physical, chemical or biochemical means such as hybridization cascades or enzymatic means. In another aspect, the optional label is a particle - oligonucleotide conjugate. The particle conjugate label comprises particles having one or more types of DNA barcodes bound directly or indirectly to the particles. The barcodes can be released as described herein and can be detected by any suitable means including arrayed substrates in a sandwich assay using any suitable detection probe. The particles may be of any suitable size including nanoparticles and micro sized particles, *e.g*., 1 µm, and may be made of any suitable material such as polymers (*e.g.,* polystyrene), metals (*e.g.,* gold or silver), ceramics, semiconductor material.

Certain biobarcode detection assays are described, for example, in U.S. Patent Application No. 10/877,750, filed June 25,2004 which is incorporated by reference in its entirety. In the case of protein detection, there are very few methods comparable to PCR that allows one to amplify the signal associated with a protein recognition event. The most promising are immuno-PCR (T. Sano, C.L. Smith, C.R. Cantor, Science 258, 120 (1992)) and the bio-bar-code amplification (Nam, J.M., Thaxton, C.S., Mirkin, C.A. (2003) Science 301, 1884-1886; and Nam, J.M. Stoeva, S.I., Mirkin, C.A. (2004) J. Am. Chem. Soc. 126, 5932-5933) approach to protein detection. The biobarcode amplification approach has the advantage that it is higher in sensitivity than immuno-PCR for the systems studied thus far, does not rely on enzymatic amplification, and is less complex. For a description of the biobarcode assay, see U.S. Patent Application No. 10/877,750, filed June 25, 2004.

As used herein, the term "particle" refers to a small piece of matter that can preferably be composed of metals, silica, silicon-oxide, or polystyrene. A "particle" can be any shape, such as spherical or rod-shaped. The term "particle" as used herein specifically encompasses both nanoparticles and microparticles as defined and described hereinbelow. Capture probes, detector probes, and barcode probes of the invention can comprise particles, which serve, for example, as carriers for antibodies, detector oligonucleotides, barcodes, and/or aptamers.

In one embodiment, a particle used in a method of the invention is a nanoparticle. Nanoparticles useful in the practice of the invention include metal (e.g., gold, silver, copper and platinum), semiconductor (e.g., CdSe, CdS, and CdS or CdSe coated with ZnS) and magnetic (e.g., ferromagnetite) colloidal materials. Other nanoparticles useful in the practice of the invention include ZnS, ZnO, TiO₂, AgI, AgBr, HgI₂, PbS, PbSe, ZnTe, CdTe, In₂ S₃, In₂ Se₃, Cd₃ P₂, Cd₃ As₂, InAs, and GaAs. The size of the nanoparticles is preferably from about 5 nm to about 150 nm (mean diameter), more preferably from about 5 to about 50 nm, most preferably from about 10 to about 30 nm. The nanoparticles may also be rods. Other nanoparticles useful in the invention include silica and polymer (e.g. latex) nanoparticles.

Methods of making metal, semiconductor and magnetic nanoparticles are well-known in the art. See, e.g., Schmid, G. (ed.) Clusters and Colloids (VCH, Weinheim, 1994); Hayat, M. A. (ed.) Colloidal Gold: Principles, Methods, and Applications (Academic Press, San Diego, 1991); Massart, R., IEEE Taransactions On Magnetics, 17, 1247 (1981); Ahmadi, T. S. et al., Science, 272, 1924 (1996); Henglein, A. et al., J. Phys. Chem., 99, 14129 (1995); Curtis, A. C., et al., Angew. Chem. Int. Ed. Engl., 27, 1530 (1988). Methods of making silica nanoparticles impregnated with fluorophores or phosphors are also well known in the art (see Tan and coworkers, PNAS, 2004, 101, 15027 - 15032).

Methods of making ZnS, ZnO, TiO₂, AgI, AgBr, HgI₂, PbS, PbSe, ZnTe, CdTe, In₂ S₃, In₂ Se₃, Cd₃ P₂, Cd₃ As₂, InAs, and GaAs nanoparticles are also known in the art. See, e.g., Weller, Angew. Chem. Int. Ed. Engl., 32, 41 (1993); Henglein, Top. Curr. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992).

Suitable nanoparticles are also commercially available from, e.g., Ted Pella, Inc. (gold), Amersham Corporation (gold) , Nanoprobes, Inc. (gold), and Quantom Dot Inc. (core-shell semiconductor particles such as CdSe/ZnS).

Nanoparticles have been a subject of intense interest owing to their unique physical and chemical properties that stem from their size. Due to these properties, nanoparticles offer a promising pathway for the development of new types of biological sensors that are more sensitive, more specific, and more cost effective than conventional detection methods. Methods for synthesizing nanoparticles and methodologies for studying their resulting properties have been widely developed over the past 10 years (Klabunde, editor, Nanoscale Materials in Chemistry, Wiley Interscience, 2001). However, their use in biological sensing has been limited by the lack of robust methods for functionalizing nanoparticles with biological molecules of interest due to the inherent incompatibilities of these two disparate materials. A highly effective method for functionalizing nanoparticles with modified oligonucleotides has been developed. See U.S. Patent Nos. 6,361,944 and 6,417,340 (assignee: Nanosphere, Inc.). The process leads to nanoparticles that are heavily functionalized with oligonucleotides, which have surprising particle stability and hybridization properties. The resulting DNA-modified particles have also proven to be very robust as evidenced by their stability in solutions containing elevated electrolyte concentrations, stability towards centrifugation or freezing, and thermal stability when repeatedly heated and cooled. This loading process also is controllable and adaptable. Such methods can also be used to generate nanoparticle-aptamer conjugates.

Nanoparticles of differing size and composition have been functionalized, and the loading of oligonucleotide recognition sequences onto the nanoparticle can be controlled via the loading process. Suitable, but non-limiting examples of nanoparticles include those described U.S. Patent No. 6,506,564; International Patent Application No. PCTIUS02/16382; U.S. Patent Application Serial No. 10/431,341 filed May 7, 2003; and International Patent Application No. PCT/US03/14100.

Nanoparticles having bound thereto aptamers and optional diluent oligonucleotides are preferably prepared by a salt aging method for preparing nanoparticle-oligonucleotide conjugates as described in U.S. Patent no. 6,506,564, Aptamers and oligonucleotides having covalently bound thereto a moiety comprising a functional group which can bind to the nanoparticles are used. The moieties and functional groups are those described in U.S. Patent nos. 6,506,564 and 6,767,702 for binding (*i*.*e*., by chemisorption or covalent bonding) oligonucleotides to nanoparticles. For instance, oligonucleotides having an alkanethiol or an alkanedisulfide covalently bound to their 5' or 3' ends can be used to bind the oligonucleotides to a variety of nanoparticles, including gold nanoparticles. Thioaptamers having phosphorothioate or phosphorodithioate functional moieties covalently bound to their 5' or 3' ends can be used to bind the aptamers to a variety of nanoparticles, including gold nanoparticles. Additionally, the oligonucleotides can be bound through an oligonucleotide tail such as a polyA tail which has a high affinity for the gold nanoparticle surface (see Tarlov and coworkers, JACS, 2004). Alternatively, streptavidin or x-biotin modified nanoparticles can be contacted with biotinylated aptamers to form the aptamer nanoparticle conjugate.

The aptamers and optional diluent oligonucleotides are contacted with the nanoparticles in water for a time sufficient to allow at least some of the aptamers and oligonucleotides to bind to the nanoparticles by means of the functional groups. Such times can be determined empirically. For instance, it has been found that a time of about 12-24 hours gives good results. Other suitable conditions for binding of the aptamers and oligonucleotides can also be determined empirically. For instance, a concentration of about 10-20 nM nanoparticles and incubation at room temperature gives good results.

Next, at least one salt is added to the water to form a salt solution. The salt can be any water-soluble salt. For instance, the salt may be sodium chloride, magnesium chloride, potassium chloride, ammonium chloride, sodium acetate, ammonium acetate, a combination of two or more of these salts, or one of these salts in phosphate buffer. Preferably, the salt is added as a concentrated solution, but it could be added as a solid. The salt can be added to the water all at one time or the salt is added gradually over time. By "gradually over time" is meant that the salt is added in at least two portions at intervals spaced apart by a period of time. Suitable time intervals can be determined empirically.

The ionic strength of the salt solution must be sufficient to overcome at least partially the electrostatic repulsion of the oligonucleotides from each other and, either the electrostatic attraction of the negatively-charged oligonucleotides for positively-charged nanoparticles, or the electrostatic repulsion of the negatively-charged oligonucleotides from negatively-charged nanoparticles. Gradually reducing the electrostatic attraction and repulsion by adding the salt gradually over time has been found to give the highest surface density of oligonucleotides on the nanoparticles. Suitable ionic strengths can be determined empirically for each salt or combination of salts. A final concentration of sodium chloride of from about 0.1 M to about 1.0 M in phosphate buffer, preferably with the concentration of sodium chloride being increased gradually over time, has been found to give good results.

After adding the salt, the aptamers, oligonucleotides and nanoparticles are incubated in the salt solution for an additional period of time sufficient to allow sufficient additional oligonucleotides to bind to the nanoparticles to produce the stable nanoparticle conjugates having aptamers and oligonucleotides bound thereto. As will be described in detail below, an increased surface density of the oligonucleotides on the nanoparticles has been found to stabilize the conjugates. The time of this incubation can be determined empirically. A total incubation time of about 24-48, preferably 40 hours, has been found to give good results (this is the total time of incubation; the salt concentration can be increased gradually over this total time). This second period of incubation in the salt solution is referred to herein as the "aging" step. Other suitable conditions for this "aging" step can also be determined empirically. For instance, incubation at room temperature and pH 7.0 gives good results.

Capture probe-nanoparticle conjugates produced by use of the "aging" step have been found to be considerably more stable than those produced without the "aging" step. As noted above, this increased stability is due to the increased density of the oligonucleotides on the surfaces of the nanoparticles which is achieved by the "aging" step. The surface density achieved by the "aging" step will depend on the size and type of nanoparticles and on the length, sequence and concentration of the aptamers/oligonucleotides. A surface density adequate to make the nanoparticles stable and the conditions necessary to obtain it for a desired combination of nanoparticles and aptamers/oligonucleotides can be determined empirically.

The aforementioned loading method for preparing DNA-modified nanoparticles, particularly aptamer-modified gold nanoparticle probes, has led to the development of a colorimetric sensing scheme for oligonucleotides and non-nucleic acid targets. See, for instance, U.S. Patent No. 6,506,564, describes a colorimetric sensing scheme based on DNA-modified nanoparticles. This method is based on the hybridization of two gold nanoparticle probes to two distinct regions of a target, e.g., DNA, of interest. Since each of the probes are functionalized with multiple oligonucleotides bearing the same sequence, the binding of the target results in the formation of target/gold nanoparticle probe aggregate when sufficient target is present. The DNA target recognition results in a colorimetric transition due to the decrease in interparticle distance of the particles. This colorimetric change can be monitored optically, with a UV-vis spectrophotometer, or visually with the naked eye. In addition, the color is intensified when the solutions are concentrated onto a membrane. Therefore, a simple colorimetric transition provides evidence for the presence or absence of a specific DNA sequence. Using this assay, femtomole quantities and nanomolar concentrations of model DNA targets and polymerase chain reaction (PCR) amplified nucleic acid sequences have been detected.

The development of DNA-modified nanoparticle conjugates, particularly aptamer-inodified gold nanoparticle probes, has also led to a colorimetric method for monitoring scattered light for oligonucleotides and non-nucleic acid targets. See U.S. Ser. No. 10/995,051, filed November 22, 2004. The scatter-based colorimetric detection method provides much higher sensitivity (> 4 orders of magnitude) in nucleic acid detection than the previously reported absorbance-based spot test when coupled to an improved hybridization method based on neutral or anionic polysaccharides that enables probe-target binding at low target concentrations. Moreover, the methods of the invention enable the detection of probe-target complexes containing two or more particles in the presence of a significant excess of non-complexed particles, which drives hybridization in the presence of low target concentrations. Also, dextran sulfate mediated probe-target complex formation in conjunction with evanescent induced scatter as provided herein enables a simple homogeneous hybridization and colorimetric detection protocol for nucleic acid sequences in total bacterial DNA, or with antibody-antigen interactions.

As described herein, nanoparticle probes, particularly gold nanoparticle probes comprising aptamer and/or antibodies, are surprising and unexpectedly suited for detection of analytes. A silver-based signal amplification procedure in a microarray-based assay can further provide ultra-high sensitivity enhancement. Silver staining can be employed with any type of nanoparticles that catalyze the reduction of silver. Preferred are nanoparticles made of noble metals (*e.g*., gold and silver). See Bassell, et al., J. Cell Biol., 126, 863-876 (1994); Braun-Howland et al., Biotechniques, 13, 928-931 (1992). Silver staining can be used to produce or enhance a detectable change in any assay performed on a substrate, including those described above. In particular, silver staining has been found to provide a huge increase in sensitivity for assays employing a single type of nanoparticle so that the use of layers of nanoparticles, aggregate probes and core probes can often be eliminated.

A nanoparticle can be detected in a method of the invention, for example, using an optical or flatbed scanner. The scanner can be linked to a computer loaded with software capable of calculating grayscale measurements, and the grayscale measurements are calculated to provide a quantitative measure of the amount of analyte detected.

Suitable scanners include those used to scan documents into a computer which are capable of operating in the reflective mode (*e.g*., a flatbed scanner), other devices capable of performing this function or which utilize the same type of optics, any type of greyscale-sensitive measurement device, and standard scanners which have been modified to scan substrates according to the invention.

The software can also provide a color number for colored spots and can generate images (*e.g*., printouts) of the scans, which can be reviewed to provide a qualitative determination of the presence of a nucleic acid, the quantity of a nucleic acid, or both. In addition, it has been found that the sensitivity of assays can be increased by subtracting the color that represents a negative result from the color that represents a positive result.

The computer can be a standard personal computer, which is readily available commercially. Thus, the use of a standard scanner linked to a standard computer loaded with standard software can provide a convenient, easy, inexpensive means of detecting and quantitating nucleic acids when the assays are performed on substrates. The scans can also be stored in the computer to maintain a record of the results for further reference or use. Of course, more sophisticated instruments and software can be used, if desired.

A nanoparticle can be detected in a method of the invention, for example, using resonance light scattering, after illumination by various methods including dark-field microscopy, evanescent waveguides, or planar illumination of glass substrates. Metal particles > 40 nm diameter scatter light of a specific color at the surface plasmon resonance frequency (Yguerabide, J.; Yguerabide, E. E. Anal. Biochem. (1998), 262, 157-176) and can be used for multicolor labeling on substrates by controlling particle size, shape, and chemical composition (Taton, T. A.; Lu, G.; Mirkin, C. A. J. Am. Chem. Soc. (2001), 123, 5164-5165; Jin, R. C.; Cao, Y. W.; Mirkin, C. A.; Kelly, K. L.; Schatz, G. C.; Zheng, J. G. Science (2001), 294, 1901-1903 )In another embodiment, a nanoparticle can be detected in a method of the invention, for example, using surface enhanced raman spectroscopy (SERS) in either a homogeneous solution based on nanoparticle aggregation (Graham and coworkers, Angew. Chem., 2000, 112, 1103.) or on substrates in a solid-phase assay (Porter and coworkers, Anal. Chem., 1999, 71, 4903-4908), or using silver development followed by SERS (Mirkin and coworkers, Science, 2002, 297, 1536-1540).

In another embodiment, the particles of the invention are detected by photothermal imaging (Boyer et. al, Science, 2002, 297, 1160-1163). In another embodiment, the particles of the invention are detected by diffraction-based sensing technology (Bailey et. al, J. Am Chem. Soc., 2003, 125, 13541). In another embodiment, the particles of the invention are detected by hyper-Rayleigh scattering (Kim et. al, Chem Phys. Lett., 2002, 352, 421).

In another embodiment, aptamers and/or antibodies attached to a substrate can be located between two electrodes, the particles can be made of a material that is a conductor of electricity, and the detecting step in the methods of the invention can comprise detecting a change in conductivity. In yet another embodiment, a plurality of aptamers and/or antibodies, each of which can recognize a different target analyte, are attached to a substrate in an array of spots and each spot of aptamers and/or antibodies is located between two electrodes, the nanoparticles are made of a material that is a conductor of electricity, and detecting step in the methods of the invention comprises detecting a change in conductivity. The electrodes can be made, for example, of gold and the nanoparticles are made of gold. Alternatively, a substrate can be contacted with silver stain to produce a change in conductivity.

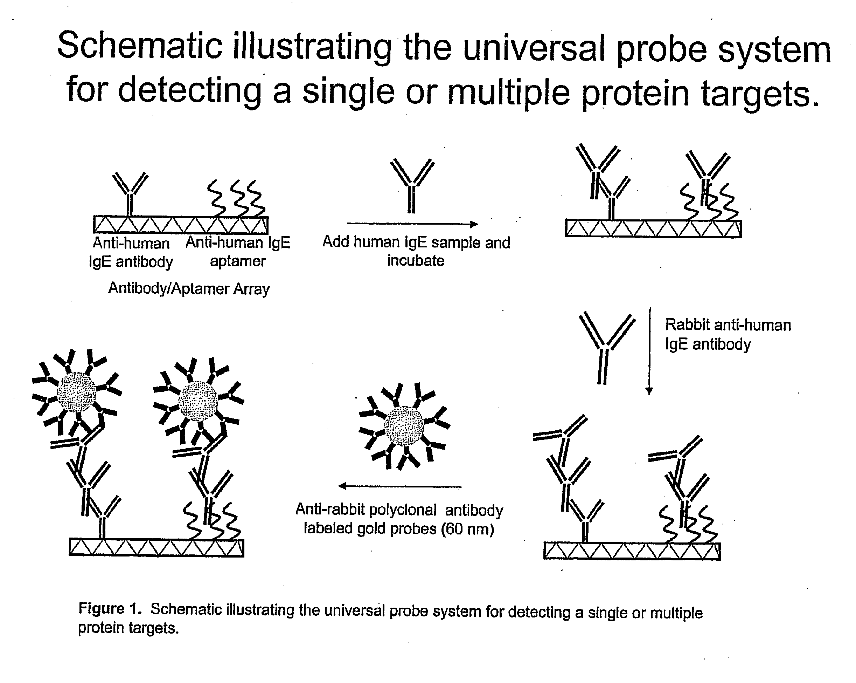

In certain embodiments, the invention provides methods for detecting a target analyte in a sample. In a particular embodiment, the method comprises a substrate that has at least one type of capture probe bound thereto. Each type of capture probe can be specific for a different target analyte (*i.e.* each probe can bind to a different analyte), or each type of capture probe can be specific for the same target analyte but comprises a different type of binding molecule (*e.g*. one type can be an aptamer and one type can be an antibody, but both aptamer and antibody can bind to the same target analyte). In particular embodiments, the methods of the invention comprise the steps of contacting the substrate with a target analyte, and then contacting the analyte with a detector probe, so that the capture probe, detector probe, and target analyte form a complex on the substrate, as illustrated, for example, in Figure 1. The complex is detectable using detection methods described herein. The presence of a complex indicates the presence of the target analyte in the sample.

In other embodiments, the substrate can have capture probes and capture oligonucleotides bound thereto. The capture probes can comprise antibodies and/or aptamers that can bind to a target analyte. Alternatively, the capture probes can comprise oligonucleotides and can hybridize to a target analyte that comprises a nucleic acid molecule.

In yet other embodiments, the methods of the invention can comprise wash steps that can be used to wash unbound materials (*e.g.* unbound probes and any other materials that could interfere with a detectable signal) from the substrate at any point prior to a detecting step.

In certain embodiments, the invention provides substrates that comprise both antibodies and aptamers bound thereto. The antibodies and aptamers can be specific for the same target analyte (*i.e*., both bind to the same target analyte) or each can be specific for a different target analyte (*i*.*e*., antibodies could bind specifically to one target analyte and the aptamers could bind to a different target analyte). A substrate can be arrayed with multiple capture probes that can bind different target analytes, thereby enabling the detection of multiple target anlaytes on a single substrate in a single assay.

In additional embodiments, the invention provides substrates that comprise capture probes (such as oligonucleotides, antibodies and/or aptamers) and capture oligonucleotides bound thereto. The capture probes can be specific for target analtyes, while the capture oligonucleotides can be specific for hybridizing to a DNA barcode. The capture probes can be specific for proteins or nucleic acid molecules. In certain aspects, the substrate can comprise multiple types of capture probes, some that bind proteins and some that bind nucleic acid molecules, thereby enabling the detection of proteins and nucleic acid molecules on a single substrate in a single assay.

In yet another embodiment of the invention, a kit for detecting for one or more analytes in a sample, the kit comprising a substrate that has: (a) at least two types of capture probes bound thereto, wherein one type of capture probe comprises antibodies and a second type of capture probe comprises aptamers or oligonucleotides; or (b) at least one type of capture probe and at least one type of detector probe bound thereto, wherein the capture probe can comprise antibodies or aptamers or oligonucleotides. The substrate may be arrayed with at least one capture probe for a specific target analyte. The kits can also comprise detector probes and barcode probes as described herein.

### EXAMPLES

The invention is demonstrated further by the following illustrative examples.

### Example 1: Detection of Human IgE on substrates containing immobilized x-IgE aptamer and x-IgE antibody

In this Example, a representative array containing an antibody and DNA aptamer (SEQ ID NO.:1) was prepared for the use in the detection of human IgE antibodies. Tasset and coworkers originally reported an aptamer oligonucleotide sequence that binds to human IgE with high affinity and high specificity (Wiegand et. al, 1996, The Journal of Immunology, Vol. 157, 221-230). Subsequently, an aptamer sequence with an extended stem-loop structure was designed to increase the IgE binding affinity (Liss et. al, 2002, Anal. Chem, Vol. 74, 4488 - 4495) and was used in this example for IgE detection. A monoclonal x-IgE antibody was purchased from OEM concepts, Inc. [Cat. #M2-G40 CLONE #090-11348] for IgE detection in this example. Codelink slides were purchased from Amersham, Inc. 60 nm diameter gold particles were manufactured by British BioCell International (BBI) [ Cat. # EM.GC60].

SEQ ID NO. 1: 5'-GCG CGG GGC ACG TTT ATC CGT CCC TCC TAG TGG
CGT GCC CCG CGC PEG PEG NH2-3'

### Preparation of arrays of immobilized x-IgE antibodies and aptamers.

The antibodies and aptamers were arrayed onto Codelink slides (Amersham, Inc.) using a GeneMachines OmniGrid accent microarrayer (Genomic Solutions, Ann Arbor, MI). The x-IgE antibodies were buffered in 1x phosphate buffered saline (pH 7.4), 60 mM trehalose at a final concentration of 500ug/mL. The x-IgE aptamers were buffered in 150mM sodium phosphate pH 8.5, 0.005% SDS. After printing, the sides were incubated overnight in a humidity chamber, and subsequently washed with TBS-T Buffer (150mM NaCl/ 10mM Tris Base buffer (pH 8) containing 0.05 % Tween20). Arrays were immersed in 1x PBS, 1mM MgCl₂, 0.01% Tween 20 and heated to 65 C for 5min, rinsed with water and spun dry. Ten sub-arrays were printed on each slide. Each sub-array contained six spots of the antibody and aptamer.

### Preparation of antibody-modified gold nanoparticles.

Polyclonal anti-rabbit antibodies were purchased from Protos Immunoresearch [Cat. # 222] (Temecula, CA). Gold nanoparticles 60 nm in diameter were purchased from British BioCell International (BBI). Briefly, 5 uL of 0.1 M sodium carbonate buffer was added to 1 mL of the 60 nm diameter gold particles to adjust the pH. Next, 10 µg of the anti-rabbit antibody was added to the buffered gold nanoparticle and incubated at room temperature for 30 minutes. The antibody-gold nanoparticle conjugates were isolated by centrifugation at 2100 G for 25 minutes. The supernatant was removed following centrifugation, and the particles were redispersed in buffer (20 mM Tris buffer (pH 7.0), 0.5 % BSA and 0.01% azide). The final nanoparticle concentration was measured by UV-visible absorbance at 520 nm using an estimated extinction coefficient of ε₅₂₀ = 2.8 x 10¹⁰ M⁻¹cm⁻¹ for the 60 nm diameter gold particles.

### Detection of Human IgE on antibody/aptamer arrays

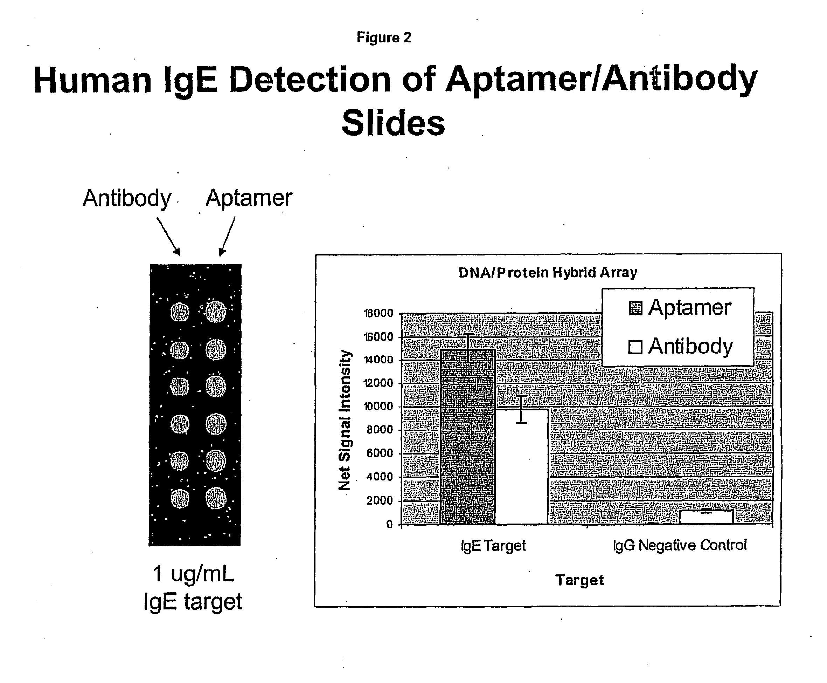

In the first step of the assay, 1 ug/mL of human IgE target [Chemicon - Cat. # A630P] or human IgG negative control [Sigma Cat. # I4506] were incubated on separate test arrays in 1X PBS buffer (pH 7.2), 0.01 % Tween 20 as assay buffer for 7 minutes at - 24°C. The slides were then washed with assay buffer and then rabbit Polyclonal x-IgE antibody (10 ug/mL in assay buffer) was added to each array for 5 minutes. Following a second wash with assay buffer, anti-rabbit antibody coated gold probe (400 pM) in the assay buffer containing 0.1% BSA, 2 % dextran sulfate was added via a pipette to each array and incubated for 3 minutes, followed by a wash with assay buffer. The slide was spun dry, rinsed with water to remove remaining salt, and spun dry again prior to imaging. Imaging was performed with an Arrayworx image analyzer (Applied Precision Inc.), Figure 2. The image data was quantified using Genepix software (Axon instruments). Both the antibody and aptamer immobilized on the array bind to human IgE target as indicated by signal at the respective array locations.

### Example 2: Detection of proteins using DNA barcodes and hybrid arrays

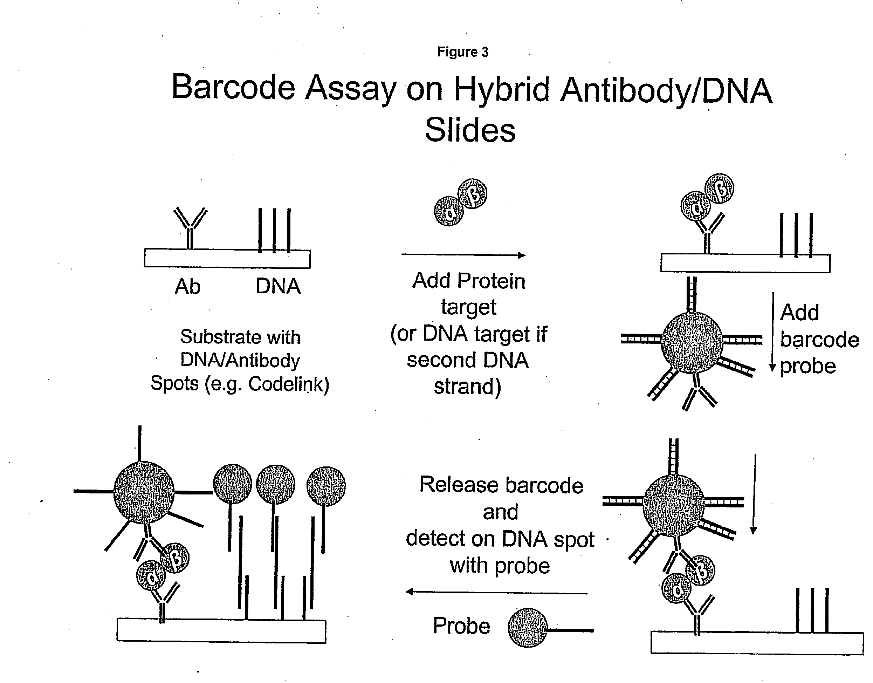

In another example not covered by the present invention, an antibody attached to a substrate recognizes a specific protein target, which is recognized by a antibody-nanoparticle conjugate with attached nucleic acid barcodes, Figure 3. The nucleic acid barcodes are released and detected on complementary nucleic acids attached on a separate portion of the substrate using a nanoparticle probe.

## Claims

1. A method for detecting at least one target analyte in a sample, the target analyte having at least two binding sites, the method comprising the steps of:
a) providing a substrate having bound thereto a first type of capture probe that comprises a target recognition element and a second type of capture probe that comprises a nucleic acid aptamer, wherein each type of capture probe can bind to a first binding site of the target analyte;
b) contacting the sample with the substrate and at least one type of detector probe under conditions that are effective for the binding of each type of capture probe to the first binding site of the target analyte and for the binding of the detector probe to a second binding site of the target analyte, wherein binding of the capture probe and the detector probe to the target analyte forms a complex;
c) washing the substrate to remove unbound materials; and
d) detecting for the presence or absence of the complex, wherein the presence or absence of the complex is indicative of the presence or absence of the target analyte in the sample, wherein the target recognition element comprises an antibody, antibody fragment, or single-chain antibody fragment (scFv), or peptides.

2. The method of claim 1, wherein the sample is first contacted with the detector probe so that the target analyte present in the sample binds to the detector probe, and the target analyte bound to the detector probe is then contacted with the substrate so that the target analyte binds to at least one type of capture probe on the substrate.

3. The method of claim 1, wherein the sample is first contacted with the substrate so that the target analyte present in the sample binds to at least one type of capture probe, and the target analyte bound to the capture probe is then contacted with the detector probe so that the target analyte binds to the detector probe.

4. The method of claim 1, wherein the sample, the detector probe and the capture probes on the substrate are contacted simultaneously.

5. The method of claim 1, wherein the captured target-detector probe complex is detected by photonic, electronic, acoustic, opto-acoustic, gravity, electxo-chemical, electro-optic, mass-spectrometric, enzymatic, chemical, biochemical, or physical means.

6. The method of claim 1, wherein the target analyte is a protein and the detector probe is a nanoparticle probe that comprises:
a) antibodies that bind the target analyte;
b) aptamers that bind the target analyte; or
c) a mixture of antibodies and aptamers that bind the target analyte.

7. The method of claim 5, wherein the nanoparticles are made of a noble metal.

8. The method of claim 7, wherein the nanoparticles are made of gold or silver.

9. The method of claim 8, wherein the nanoparticles are made of gold.

10. The method of claim 1, wherein the detector probe comprises fluorophores, phosphors, quantum dots, enzyme conjugates, or antibody DNA conjugates.

11. The method of claim 1, wherein the substrate is a nanoparticle, a thin film, or a magnetic bead.

12. The method of claim 1, wherein the substrate has a planar surface.

13. The method of claim 1, wherein the substrate is made of glass, quartz, ceramic, or plastic.

14. The method of claim 1, wherein the detecting comprises contacting the substrate with silver stain.

15. The method of claim 1, wherein the detector probes comprise nanoparticles and wherein the step of detecting comprises detecting light scattered by the nanoparticles.

16. The method of claim 1, wherein the substrate is addressable.

17. The method of claim 16, wherein a plurality of capture probes, each of which can recognize a different target analyte, are attached to the substrate in an array of spots.

18. The method according to claim 17, wherein each spot of capture probes is located between two electrodes, and wherein the detector probes comprise nanoparticles made of a material that is a conductor of electricity, and the detection step comprises detecting a change in conductivity.

19. The method of claim 18, wherein the electrodes are made of gold and the nanoparticles are made of gold.

20. The method of claim 18, wherein the substrate is contacted with silver stain to produce the change in conductivity.

21. A substrate having bound thereto antibodies and nucleic acid aptamers, wherein the substrate is a nanoparticle or a magnetic bead and the antibodies and aptamers are specific for the same target analyte.

## Patentansprüche

1. Verfahren zur Detektion wenigstens eines Zielanalyten in einer Probe, wobei der Zielanalyt wenigstens zwei Bindungsstellen besitzt, das Verfahren umfassen die Schritte:
a) Zur Verfügung stellen eines Substrats, das daran gebunden eine erste Art einer Fangsonde, welche ein Zielerkennungselement umfasst, besitzt, und eine zweite Art einer Fangsonde, die ein Nukleinsäureaptamer umfasst, wobei jede Art Fangsonde an eine erste Bindungsstelle des Zielanalyten binden kann;
b) In Kontakt bringen der Probe mit dem Substrat und wenigstens einer Art Detektorsonde unter Bedingungen, die die Bindung jeder Art Fangsonde an die erste Bindungsstelle des Zielanalyten und die Bindung der Detektorsonde an eine zweite Bindungsstelle des Zielanalyten ermöglichen, wobei die Bindung der Fangsonden und der Detektorsonde an den Zielanalyten zur Bildung eines Komplexes führt;
c) Waschen des Substrats, um ungebundene Materialien zu entfernen; und
d) Detektion der Anwesenheit oder Abwesenheit des Komplexes, wobei die Anwesenheit oder Abwesenheit des Komplexes die Anwesenheit oder Abwesenheit des Zielanalyten in der Probe anzeigt, wobei das Zielerkennungselement einen Antikörper, ein Antikörperfragment oder einzelkettiges Antikörperfragment (scFv) oder Peptide umfasst.

2. Das Verfahren von Anspruch 1, wobei die Probe erst mit der Detektorsonde in Kontakt gebracht wird, so dass der in der Probe vorhandene Zielanalyt an die Detektorsonde bindet, und der an die Detektorsonde gebundene Zielanalyt wird dann mit dem Substrat in Kontakt gebracht, so dass der Zielanalyt an wenigstens eine Art Fangsonde auf dem Substrat bindet.

3. Das Verfahren von Anspruch 1, wobei die Probe erst mit dem Substrat in Kontakt gebracht wird, so dass der in der Probe vorhandene Zielanalyt an wenigstens eine Art Fangsonde bindet, und der an die Fangsonde gebundene Zielanalyt wird dann mit der Detektorsonde in Kontakt gebracht, so dass der Zielanalyt an die Detektorsonde bindet.

4. Das Verfahren von Anspruch 1, wobei die Probe, die Detektorsonde und die Fangsonden auf dem Substrat gleichzeitig in Kontakt gebracht werden.

5. Das Verfahren von Anspruch 1, wobei der eingefangene Ziel-Detektorsondenkomplex durch Photonen-, elektronische, akustische, optoakustische, Schwerkraft-, elektrochemische, elektrooptische, massenspektrometrische, enzymatische, chemische, biochemische oder physikalische Mittel detektiert wird.

6. Das Verfahren von Anspruch 1, wobei der Zielanalyt ein Protein ist und die Detektorsonde eine Nanopartikelsonde ist, die umfasst:
a) Antikörper, die den Zielanalyten binden;
b) Aptamere, die den Zielanalyten binden; oder
c) eine Mischung von Antikörpern und Aptameren, die den Zielanalyten binden.

7. Das Verfahren von Anspruch 5, wobei die Nanopartikel aus einem Edelmetall hergestellt sind.

8. Das Verfahren von Anspruch 7, wobei die Nanopartikel aus Gold oder Silber hergestellt sind.

9. Das Verfahren von Anspruch 8, wobei die Nanopartikel aus Gold hergestellt sind.

10. Das Verfahren von Anspruch 1, wobei die Detektionssonde Fluorophore, Phosphore, Quantum Dots, Enzymkonjugate oder Antikörper DNA Konjugate umfasst.

11. Das Verfahren von Anspruch 1, wobei das Substrat ein Nanopartikel, ein dünner Film oder ein magnetisches Kügelchen ist.

12. Das Verfahren von Anspruch 1, wobei das Substrat eine ebene Oberfläche besitzt.

13. Das Verfahren von Anspruch 1, wobei das Substrat aus Glas, Quarz, Keramik oder Plastik hergestellt ist.

14. Das Verfahren von Anspruch 1, wobei die Detektion das in Kontakt bringen des Substrats mit Silberfärbung umfasst.

15. Das Verfahren von Anspruch 1, wobei die Detektorsonde Nanopartikel umfasst und wobei der Schritt der Detektion die Detektion von durch die Nanopartikel gestreutem Licht umfasst.

16. Das Verfahren von Anspruch 1, wobei das Substrat adressierbar ist.

17. Das Verfahren von Anspruch 16, wobei eine Vielzahl von Fangsonden, von denen jede einen anderen Zielanalyten erkennen kann, an das Substrat in einer Anordnung von Punkten gebunden ist.

18. Das Verfahren gemäß Anspruch 17, wobei jeder Punkt von Fangsonden zwischen zwei Elektroden liegt, und wobei die Detektorsonden Nanopartikel umfassen, die aus einem Material hergestellt sind, das Elektrizität leitet, und der Detektionsschritt die Detektion einer Änderung der Leitfähigkeit umfasst.

19. Das Verfahren von Anspruch 18, wobei die Elektroden aus Gold hergestellt sind und die Nanopartikel aus Gold hergestellt sind.

20. Das Verfahren von Anspruch 18, wobei das Substrat mit Silberfärbung in Kontakt gebracht wird, um die Änderung der Leitfähigkeit hervorzurufen.

21. Ein Substrat, das daran gebundene Antikörper und Nukleinsäure-Aptamere besitzt, wobei das Substrat ein Nanopartikel oder ein magnetisches Kügelchen ist und die Antikörper und Aptamere spezifisch für den gleichen Zielanalyten sind.

## Revendications

1. Procédé de détection d'au moins un analyte cible dans un échantillon, l'analyte cible ayant au moins deux sites de liaison, le procédé comprenant les étapes consistant à :
a) fournir un substrat ayant liés à celui-ci un premier type de sonde de capture qui comprend un élément de reconnaissance de cible et un second type de sonde de capture qui comprend un aptamère d'acide nucléique, où chaque type de sonde de capture peut se lier à un premier site de liaison de l'analyte cible ;
b) mettre l'échantillon en contact avec le substrat et au moins un type de sonde détecteur dans des conditions qui sont efficaces pour la liaison de chaque type de sonde de capture au premier site de liaison de l'analyte cible et pour la liaison de la sonde détecteur à un second site de liaison de l'analyte cible, où la liaison de la sonde de capture et de la sonde détecteur à l'analyte cible forme un complexe ;
c) laver le substrat pour éliminer les matériaux non liés ; et
d) détecter la présence ou l'absence du complexe, où la présence ou l'absence du complexe indique la présence ou l'absence de l'analyte cible dans l'échantillon, où l'élément de reconnaissance de la cible comprend un anticorps, un fragment d'anticorps ou un fragment d'anticorps à chaîne unique (scFv) ou des peptides.

2. Procédé selon la revendication 1, dans lequel l'échantillon est d'abord mis en contact avec la sonde détecteur de telle manière que l'analyte cible présent dans l'échantillon se lie à la sonde détecteur, et l'analyte cible lié à la sonde détecteur est ensuite mis en contact avec le substrat de telle manière que l'analyte cible se lie à au moins un type de sonde de capture sur le substrat.

3. Procédé selon la revendication 1, dans lequel l'échantillon est d'abord mis en contact avec le substrat de telle manière que l'analyte cible présent dans l'échantillon se lie à au moins un type de sonde de capture, et l'analyte cible lié à la sonde de capture est ensuite mis en contact avec la sonde détecteur de telle manière que l'analyte cible se lie à la sonde détecteur.

4. Procédé selon la revendication 1, dans lequel l'échantillon, la sonde détecteur et les sondes de capture sur le substrat sont mis en contact simultanément.

5. Procédé selon la revendication 1, dans lequel le complexe cible capturée-sonde détecteur est détecté par un moyen photonique, électronique, acoustique, opto-acoustique, de gravité, électrochimique, électro-optique, spectrométrique de masse, enzymatique, chimique, biochimique ou physique.

6. Procédé selon la revendication 1, dans lequel l'analyte cible est une protéine et la sonde détecteur est une sonde nanoparticulaire qui comprend :
a) des anticorps qui lient l'analyte cible ;
b) des aptamères qui lient l'analyte cible ; ou
c) un mélange d'anticorps et d'aptamères qui lient l'analyte cible.

7. Procédé selon la revendication 5, dans lequel les nanoparticules sont faites d'un métal noble.

8. Procédé selon la revendication 7, dans lequel les nanoparticules sont faites d'or ou d'argent.

9. Procédé selon la revendication 8, dans lequel les nanoparticules sont faites d'or.

10. Procédé selon la revendication 1, dans lequel la sonde détecteur comprend des fluorophores, des phosphores, des points quantiques, des conjugués enzymatiques ou des conjugués anticorps ADN.

11. Procédé selon la revendication 1, dans lequel le substrat est une nanoparticule, un film mince ou une bille magnétique.

12. Procédé selon la revendication 1, dans lequel le substrat a une surface planaire.

13. Procédé selon la revendication 1, dans lequel le substrat est fait de verre, de quartz, de céramique ou de plastique.

14. Procédé selon la revendication 1, dans lequel la détection comprend la mise en contact du substrat avec un colorant à l'argent.

15. Procédé selon la revendication 1, dans lequel les sondes détecteurs comprennent des nanoparticules et dans lequel l'étape de détection comprend la détection de la lumière diffusée par les nanoparticules.

16. Procédé selon la revendication 1, dans lequel le substrat est contrôlable.

17. Procédé selon la revendication 16, dans lequel une pluralité de sondes de capture, dont chacune peut reconnaître un analyte cible différent, sont fixées au substrat en un réseau de points.

18. Procédé selon la revendication 17, dans lequel chaque point des sondes de capture est localisé entre deux électrodes, et dans lequel les sondes détecteurs comprennent des nanoparticules faites d'un matériau qui est conducteur de l'électricité, et l'étape de détection comprend la détection d'un changement de conductivité.

19. Procédé selon la revendication 18, dans lequel les électrodes sont faites d'or et les nanoparticules sont faites d'or.

20. Procédé selon la revendication 18, dans lequel le substrat est mis en contact avec un colorant à l'argent pour produire le changement de conductivité.

21. Substrat ayant liés à celui-ci des anticorps et des aptamères d'acide nucléique, où le substrat est une nanoparticule ou une bille magnétique et les anticorps et les aptamères sont spécifiques du même analyte cible.
